# EUROPEAN PATENT SPECIFICATION

(11) **EP 2 744 451 B1**
(45) Date of publication and mention of the grant of the patent: **20.09.2017**
(21) Application number: 12750883.6
(22) Date of filing: 20.08.2012
(51) Int. Cl.: A61F 2/16

(54) **INTRAOCULAR LENS**
INTRAOKULARLINSE
LENTILLE INTRAOCULAIRE

(30) Priority: 19.08.2011 NL 2007285; 22.08.2011 US 201161525913 P
(43) Date of publication of application: 25.06.2014
(73) Proprietor: Oculentis Holding B.V., 6961 LZ Eerbeek (NL)
(72) Inventor: WANDERS, Bernardus Franciscus Maria, 6986 AH Angerlo (NL)
(74) Representative: Nederlandsch Octrooibureau
(86) International application number: PCT/NL2012/050579
(87) International publication number: WO 2013/028068

(56) References cited:
- EP-A1- 2 219 065
- WO-A1-2010/079537
- WO-A2-2005/055875
- DE-A1-102007 002 885
- US-A1- 2009 195 748

## Description

### Field of the Invention

The present invention relates to an intraocular lens for insertion into the human eye and to an intraocular lens as an additional lens to the natural eye lens or to an intraocular lens replacing the natural eye lens.

### Background of the Invention

The human eye in its simplest form functions to provide vision by transmitting and refracting light through a clear outer portion called the cornea, and further focussing the image by the lens onto the retina at the back of the eye. The quality of the focussed image depends on many factors including the size, shape and length of the eye, and the shape and transparency of the cornea and lens.

When trauma or disease cause the lens to become less transparent, vision deteriorates because of the diminished light which becomes transmitted to the retina. This deficiency in the lens of the eye is known as cataract. The treatment for this condition is surgical removal of the lens and implantation of an artificial lens, also known as intraocular lens (IOL). Unsatisfactory placement of the IOL, biometric errors, presbyopia or degradation of the eye cannot be readily ameliorated by lens replacement.

To correct for these refractive errors it is also known to introduce an IOL in the anterior chamber of the eye in front of the iris, for example, the Artisian iris claw lens by Ophtec BV. These and other anterior chamber phakic lenses are disclosed in US 5,071,432, US 5,192,319, US 5,300,117, US 5,928,282 and WO 98/56315.

EP 2 219 065 A1 discloses optical configurations of the optic of an IOL in which one or more additional surface sections are provided on an anterior side at a distance from the optical axis in addition to a main anterior surface section. The additional surface section(s) provide for an optical power that is different from the base optical power of the main lens formed by the main anterior surface section and the posterior surface. Transitions are present between main anterior surface section and additional anterior surface section(s), which will generally be recessed with respect to the main anterior surface section. Especially near the circumferential edge or rim of the IOL this gives rise to a considerable transition height. The generally concave nature of the transition, which might be quite steep, directs light refracted at the transition within the IOL towards its circumferential edge, where it may be internally reflected and directed towards the optical axis. Such internal reflections will present a (semi-circular) halo to a person having the IOL in his or her eye. Such effects are quite disturbing and undesired.

Further, if such a known IOL would be used as an (add-on) IOL in the sulcus or anterior chamber of the human eye it would present a surface structure on its anterior side. This may provide degradation of the iris sliding over a surface structure on the anterior side when the IOL is positioned in the sulcus, even though the anterior presents a continuous non-pinching surface to the iris.

The known IOL having the additional surface section on the anterior side can be tailored to individual requirements by machining the posterior side having no surface structure. In case of providing a strong toric component to the posterior side would yield an IOL having very steep slopes at the posterior side. Such a steeply sloped posterior side may come into conflict parts of the human eye on the posterior side of the IOL or another IOL present at the posterior side. It will provide thick sides of the IOL. It may further provide an IOL with a very thin centre such that the IOL may become mechanically unstable.

It is known to introduce a secondary lens in the sulcus. The intraocular lens is placed behind the iris in front of and outside the lens capsule. The haptics of the IOL are sandwiched between the iris and the zonules, in the region of the ciliary sulcus, to hold the lens in place. Such a secondary or add-on IOL has been disclosed, for example, in WO 2008/065362 A1. These lenses are intended for secondary implantation and the simultaneous correction of residual ametropia. It comprises a convex-concave optic wherein both faces of the optic are curved in the same sense with respect to a plane defined by the rim of the optic. The haptics for holding the IOL generally also lie in this plane. According to WO 2008/065362 A1 the angulation of the haptics is intended to minimise or prevent uveal contact with the lens. However, the IOL will always be in contact with some parts of the human eye. Contact of the anterior side of the IOL with the iris is difficult or even impossible to prevent when positioned in the sulcus, as is clearly seen in figure 2 of WO 2008/065362 A1. When such IOL would be positioned in the anterior chamber the haptics could be shaped such that the posterior side is not in contact with the iris, but then the anterior side of the IOL is likely to be in contact with the cornea.

DE 10 2007 002 885 A1 discloses an add-on IOL with haptics for holding the IOL within the eye. The document discloses that the transition of haptics to optic should not present any steps to prevent pinching of the iris. Such steps arise in the IOL disclosed since it is assembled by insertion of wire-like haptics in the circumferential surface of the optic. To not present a step in the transition of haptic to anterior side of the optic the haptic should be inserted at the edge of the anterior side and the circumferential surface. The transition will still show an edge-like curvature. The document discloses that such a transition should be smooth (continuous), but it will still present surface structure since height and/or direction differences are present, which will be damaging to the iris.

Much attention has been given to the shape of the haptics to avoid, for instance, excessive pressure or prevent rotation, but not to the specific optic configuration needs for these kind of add-on IOLs for placement in, for instance, the sulcus or the anterior chamber, especially to correct presbyopia and residual refractive errors.

### Summary of the Invention

The invention overcomes these shortcomings of the known intraocular lenses by providing an IOL (intraocular lens) for insertion into the human eye according to the claims. Said IOL comprises an optic, said optic comprising a posterior surface for facing towards the posterior chamber of the human eye; an anterior surface for facing away from the posterior chamber of the human eye, said anterior surface being at least substantially convex; a main lens formed by corresponding surface sections of said anterior and posterior surfaces, said main lens having an optical axis and a base optical power; and an additional surface section at a distance from said optical axis, said additional surface section comprising at least one additional section part, the or each additional section part providing for an optical power different from said base optical power, wherein said posterior surface is substantially flat or at least substantially concave, and said posterior surface comprises a substantially flat or at least substantially concave main posterior surface section, said main lens being formed by said anterior surface and said main posterior surface section; and said additional surface section providing an additional posterior surface section at a distance from said optical axis, said additional posterior surface section comprising said at least one additional section part providing at least one refractive posterior section part, the or each posterior section part providing for said optical power different from said base optical power.

Such an IOL is suitable to be used as an additional lens within the human eye and provides for a multifocal lens that can assist in its improved functioning. The IOL can be efficiently produced by moulding, especially the complex posterior surface of the IOL. The anterior surface can also be formed by moulding followed by a rotational machining step to produce a specific shape to the anterior surface for the IOL so that it can correct optical errors of the eye of a specific individual, if required. Production of the posterior surface is most difficult. Standard IOLs can therefore efficiently be produced and kept in stock, while it can then be tailored to specific requirements by an additional machining step of the anterior surface at a later moment in time. Such methods are not practically feasible with known IOLs.

Said additional posterior surface section can be efficiently incorporated in said posterior surface by having it at least substantially recessed with respect to said main posterior surface.

It can further be advantageous to have the anterior surface at least substantially smooth. Especially when the IOL is provided in the sulcus of the eye, the iris will experience a smooth surface and avoid to become degraded. Degradation of the iris through loss of pigment occurs when sliding over a rough or corrugated, which will cause optical artefacts in the eye.

In an embodiment said main lens has a base optical power configured for correcting a refractive error in the eye for distance vision, said base optical power being between -15 and +15 dioptre, in an embodiment between -8 and +8 dioptre. In a further embodiment the or each posterior section part has an additional optical power configured for correcting near or intermediate vision, said additional optical power of said posterior section part being between 0 and +6 dioptre, in an embodiment between 0 and +4.0 dioptre, in an embodiment between +1.5 and +4.0 dioptre, relative to said base optical power. In yet a further embodiment said additional optical power of said posterior section part varies between a minimum and a maximum value throughout said posterior section part.

In an embodiment said additional posterior surface section comprises at least one refractive posterior section part. In another embodiment said additional posterior surface section comprises at least one diffractive posterior section part. In yet another embodiment said additional posterior surface section comprises a refractive posterior surface section part and a diffractive posterior section part, said diffractive posterior section part being superimposed on said refractive posterior section part. In a further embodiment said additional posterior surface section comprises physically separated posterior section zones.

In an embodiment said optic comprises a central section within a circumscribing circle having a diameter between 0.1 and 2 mm, said optical axis of said main lens passing through said central part, said central part having an optical power between -2.0 and +2.0 dioptre, in an embodiment between -1.0 and +1.0 dioptre, relative to said base optical power. In a further embodiment said optical axis of said main lens passes through a centre of said circumscribing circle of said central section.

In an advantageous embodiment a transition zone between said main posterior surface and said additional posterior surface section is shaped such as to refract light away from said optical axis when said IOL is illuminated via a collimated lens and placed in an ISO model eye having a 4 mm diameter aperture, in an embodiment said transition zone having a curvature resulting in a loss of light within a circle with a diameter of 4 mm around said optical axis of less than about 25%, said loss of light being defined as the fraction of the amount of in-focus light from said IOL compared to the amount of light from an identical IOL without said additional posterior surface section.

In yet another embodiment said optical axis is directed such that that said optical axis is substantially aligned with an optical axis of the human eye when said IOL is provided in the human eye.

In a specific embodiment the IOL is an add-on IOL for insertion into the human eye in addition to the natural eye lens or in addition to a replacement IOL replacing the natural eye lens, said add-on IOL being an IOL as described above. In an embodiment the add-on IOL is for insertion in the sulcus between the iris and the capsular bag; or in the anterior chamber. In an advantageous embodiment such add-on IOL for insertion in the sulcus comprises haptics for holding said IOL within the human eye, said haptics enclosing an angle with a plane through a rim of said optic such that said haptics are at a side of said plane corresponding to said anterior surface of said IOL, in an embodiment an absolute value of said angle being between 5 and 15 degrees, in an embodiment between 8 and 12 degrees. In another advantageous embodiment such add-on IOL for insertion in the anterior chamber comprises haptics for holding said IOL within the human eye, said haptics enclosing an angle with a plane through a rim of said optic such that said haptics are at a side of said plane corresponding to said posterior surface of said IOL, in an embodiment an absolute value of said angle being between 5 and 15 degrees, in an embodiment between 8 and 12 degrees.

Further advantages and advantageous embodiments will become apparent from the description of embodiments of the invention.

### Brief Description of the Drawings

Embodiments of the invention will be described with reference to the drawings, in which identical or like reference numerals refer to identical or like parts, and in which
Figure 1 shows a cross-section of the human eye with an additional or add-on IOL according to the invention positioned in the sulcus;
Figure 2 shows a cross-section of the human eye with an additional or add-on IOL according to the invention positioned in the anterior chamber;
Figure 3 shows a cross-section of the human with the natural eye lens replaced by an IOL, and with an add-on IOL according to the invention positioned in the sulcus;
Figure 4 shows a front side view of an embodiment of an IOL according to the invention;
Figure 5a shows a side view of the IOL of figure 4, with a positive haptic angle;
Figure 5b shows a side view of the IOL of figure 4, with a negative haptic angle;
Figure 6 shows a cross-section of an embodiment of an IOL according to the invention along a line indicated by A-A in figure 4;
Figure 7 shows a back side view of an embodiment of an IOL according to the invention;
Figure 8 shows a back side view of another embodiment of an IOL according to the invention; Figure 9 shows a back side view of yet another embodiment of an IOL according to the invention;
Figure 10a shows a ray trace at a transition between recessed additional surface section and main surface section on the posterior surface of an IOL according to the invention; and

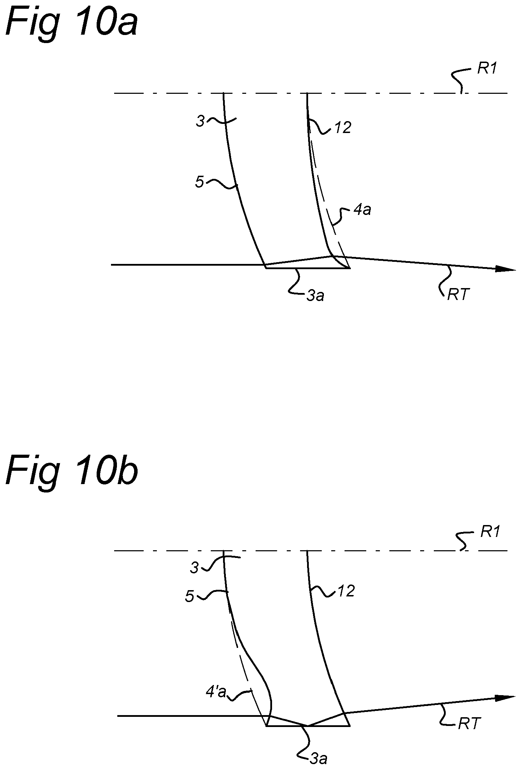

Figure 10a shows a ray trace at a transition between recessed additional surface section and main surface section on the anterior surface of a known IOL.

### Detailed Description of Embodiments

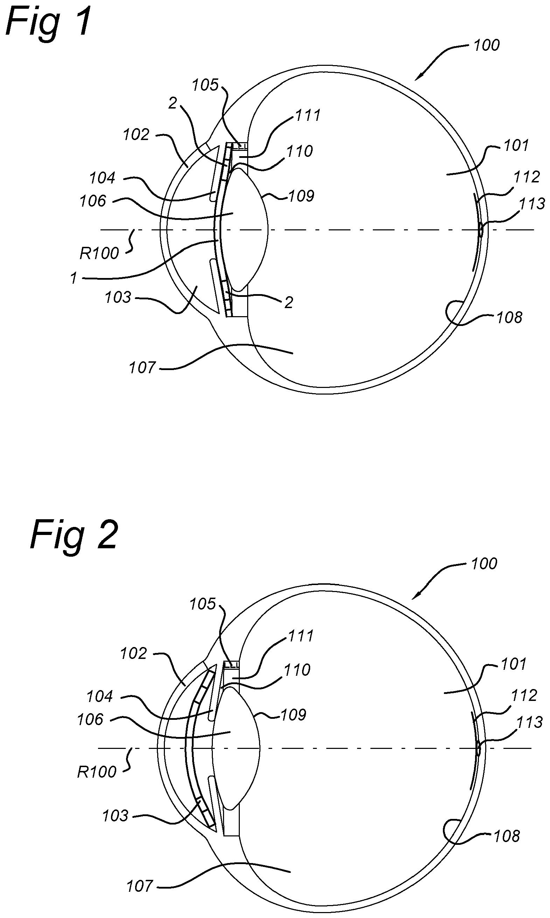

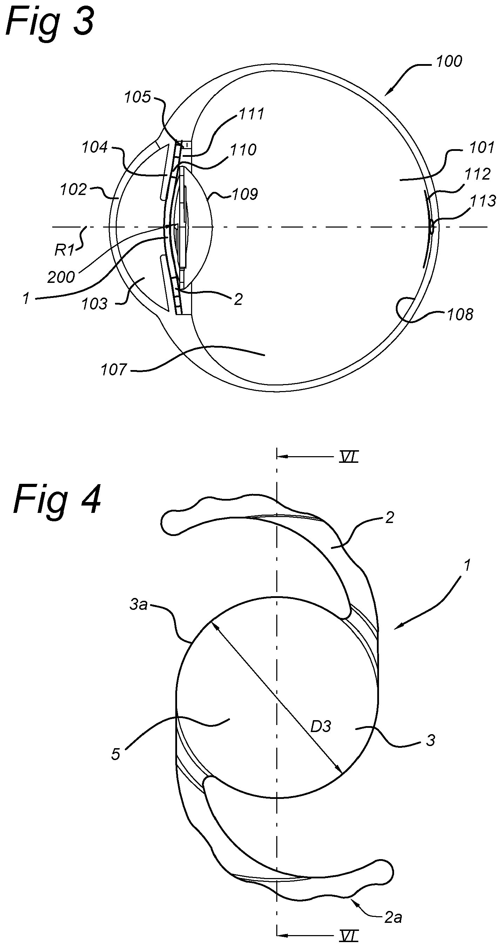

A schematic view of a human eye 100 with its natural lens 106 inside lens capsule or capsular bag 109 is shown in figure 1. The eye has a vitreous body 101 within posterior chamber 107. Retina 108 is on the inside of the posterior chamber 107, which further comprises macula 112 with fovea 113 at the centre thereof. An optical axis R100 of eye 100 passes through the centre of iris 104. The eye further has a cornea 102 and an anterior chamber 103. The lens 106 and capsular bag 109 are held by ciliary muscle 105 and zonule fibres or ciliary zonules 111. The (ciliary) sulcus 110 is in between iris 104 and ciliary zonules 111. Figure 1 further shows schematically an add-on IOL 1 according to the invention in addition to the natural eye lens 106. Add-on IOL 1 is positioned in the sulcus 110 of the eye in between iris 104 and capsular bag 109. An add-on IOL 1 according to the invention within anterior chamber 103 in addition to a natural eye lens 106 is schematically shown in figure 2. Figure 3 schematically shows an IOL 200 replacing the original lens 106 within capsular bag 109. An add-on IOL 1 is provided in addition to IOL 200 within the sulcus 110. Both IOLs have an optical axis R1 coinciding with the optical axis R100 of the human eye.

Figure 4 shows a front view on an IOL 1 according to the invention. IOL 1 comprises optic 3 and haptics 2 for holding the IOL within the human eye. Haptics 2 may have radial protrusions or ridges 2a or any other means workable to prevent rotation of IOL 1 when provided in the human eye. Especially a toric embodiment, in which the IOL is adapted to correct for any cylindrical imaging errors of the eye, requires an accurate rotational positioning of the IOL. Optic 3 has a rim 3a and a diameter D3 between 5 and 7.5 mm, in an embodiment 6.5 mm. The diameter D1 of the IOL 1 is between 12 and 14.5 mm, in an embodiment between 13 and 14 mm.

The front side of the optic 3 has an anterior surface 5 which faces away from the posterior chamber 107 when IOL 1 is provided in the human eye 100. Anterior surface 5 has an at least substantially convex shape, which means that anterior surface 5 is curved outward with respect to a plane through rim 3a.

In an embodiment the anterior surface is fabricated at least substantially smooth. This is especially advantageous when the IOL is provided in the sulcus 110 of the eye 100, as is shown in figures 1 and 3. The iris 104 will then glide smoothly over anterior surface 5 and no pigment will be removed from the iris when gliding over anterior surface 5 of IOL 1. Pigment removed from the iris may lead to unwanted visual artefacts, which will be prevented when having a smooth anterior surface of the IOL.

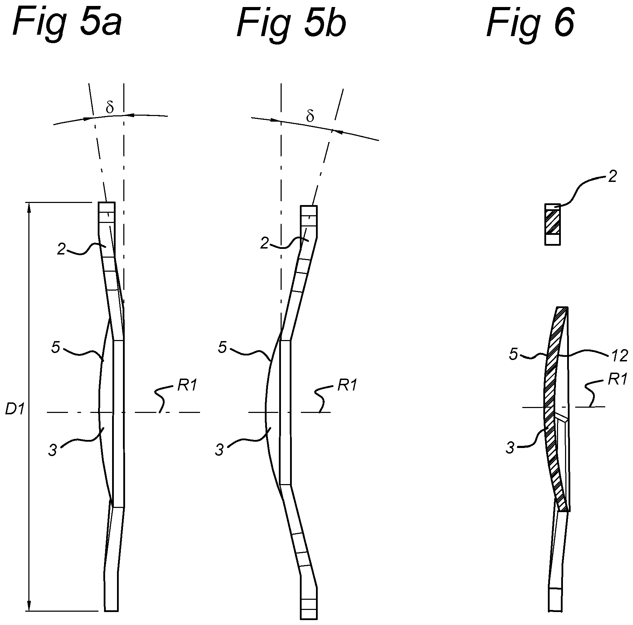

Figures 5a and 5b shows a side view of the IOL of figure 4. It is shown that haptics 2 make an angle δ with respect to a plane through rim 3a of optic 3. Such an angle δ provides a optimal placement of IOL 1 with respect to, for instance, the iris 104 when the IOL is positioned in the sulcus 110 or the anterior chamber 103. In case of placement of the IOL in the sulcus, angle δ is generally positive, i.e. haptics 120 are on the anterior surface side of optic 3 with respect to a plane through rim 3a, as is shown in figure 5b. In case of placement of the IOL in the anterior chamber 103 of the eye 100, angle δ is generally negative, so haptics 2 will be on the other side of a plane through rim 3a, as is shown in figure 5b. The absolute value of angle δ is in both cases between 5 and 15 degrees, preferable between 8 and 12 degrees.

Anterior surface 5 can have a convex spherical shape to correct for any spherical refractive deficiencies that may be present in the eye. It further may have an aspherical shape to correct for any other refractive deficiencies that may be present in the eye, to correct for spherical aberration caused by a regular cornea shape, and/or to correct for higher order optical errors caused by the irregular cornea, for instance, after laser treatment or keratoconus. Anterior surface 5 may also have a toric shape to correct for any cornea astigmatism that may be present.

Figure 6 depicts a cross-section VI-VI through the IOL as indicated in figure 4 and shows that posterior surface 12 on the back side of the IOL has a substantially concave shape. The posterior surface 12 of optic 3 faces the posterior chamber 107 when IOL 1 is positioned in the eye 100.

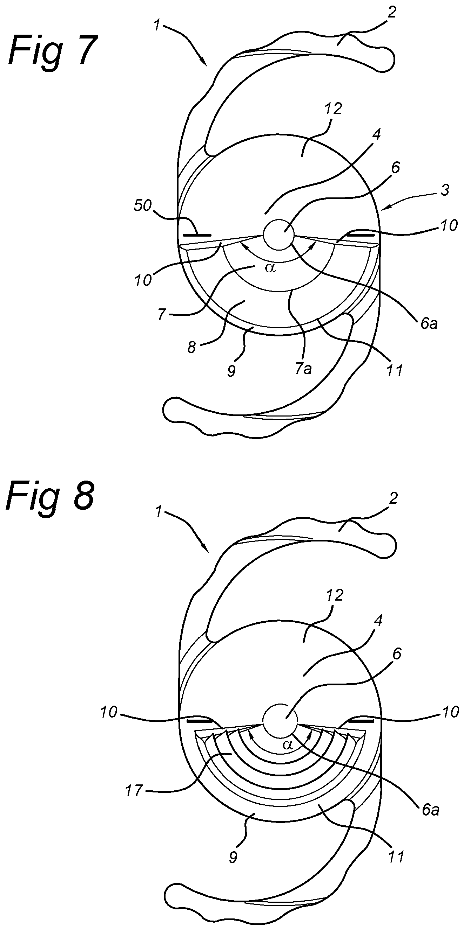

Figure 7 shows the back side of IOL 1 with optic 3 and haptics 2. Posterior surface 12 has various sections.

Posterior surface 12 comprises a substantially concave main posterior surface section 4. A main lens of optic 3 of the IOL is formed by both the substantially convex anterior surface 5 and the substantially concave main posterior surface section 4. In an embodiment the main posterior surface section 4 is corrected for spherical aberration. In general, the main lens is intended and configured for correcting the remaining refractive error for distance vision. A base optical power of the main lens may be in between -15 and +15 dioptre, preferably in between -8 and +8 dioptre. The main lens has an optical axis R1. Optical axis R1 passes through an optical centre of the main lens.

In the embodiment shown, posterior surface 12 also comprises a central section 6 that lies within a circumscribing circle 6a having a diameter between 0.1 and 2 mm. In the embodiment shown the outer boundary of central section 6 is identical to the circumscribing circle 6a shown. In an embodiment the outer boundary of central section 6 need not be circular but lies within such a circumscribing circle. The outer boundary may have any shape, but is in an embodiment substantially circular. Optical axis R1 actually passes through central section 6. The optical power of the central section is in between -2.00 and +2.00 dioptre relative to the base optical power of the main lens of optic 3 of the IOL.

Posterior surface 12 further comprises an additional posterior surface section at a distance from the optical axis R1. In the embodiment shown in figure 7 the boundary of the additional posterior surface section at a distance from optical axis R1 is denoted with reference numeral 6a. In general, the additional posterior surface section at its boundary near optical axis R1 will adjoin to either main posterior surface section 4 or central section 6. In the embodiment shown in figure 7, the additional posterior surface section comprises two section zones or section parts 7 and 8 that are physically separated by boundary or transition zone 7a.

An additional first sector zone 7 is configured for near or intermediate vision, for instance, for reading. The curvature of section zone 7 is embedded or recessed within the curvature of substantially concave main posterior surface 4. In general, the curvature of zone 7 is more flat then the curvature of main posterior surface 4. A maximum optical power of zone 7 relative to the base optical power can be +6.0 dioptre, in an embodiment between +1.50 and +4.0 dioptre. The optical power of the additional first sector zone 7 can vary across the zone between 0 and 100% of its maximum optical power. Its optical power may vary according to a specific profile in a radial direction, and may both increase and decrease in that direction. Boundary 7a is generally at a distance between 1.2 and 2.8 mm, in an embodiment between 1.5 and 2.5 mm from the boundary near optical axis R1.

Additional second sector zone 8 is also configured for either near or intermediate vision in the embodiment shown. Its curvature is also embedded or recessed within the curvature of substantially concave main posterior surface 4, and, in general, the curvature of zone 8 is more flat then the curvature of main posterior surface 4. The maximum optical power of zone 8 can be +6.0 dioptre, in an embodiment between 0 and +4.0 dioptre. Also in zone 8 the optical power may vary according to a specific profile across the zone. It can vary in a radial direction, and can both increase and decrease in that direction. The specific profile of the optical power can extend across both additional sector zones 7 and 8. The variation of the optical power across zone 8 can be between 0 and 100% of the maximum optical power of the zone. Again, the optical power of zone 8 is relative to the base optical power. Boundary or transition zone 11 physically separates additional second sector zone 8 from concentric region 9, which generally will be an extension of concave main posterior surface section 4. A distance between boundary 11 and the boundary near optical axis R1 of section 7 can be between 1.3 and 3.1 mm, in an embodiment between 2.4 and 3.0 mm.

The combined curvature of the sections 4, 6, 7, 8 and 9 of posterior surface 8 is an at least substantially concave profile. Section 9 as such can be a transition section and may as such have a shape deviating from a concave profile.

The boundaries or transition zones 6a, 7a and 11 are visible in figure 7, but need not be visible in an actual IOL. Transition zone 6a, 7a and 11 may be configured such as to fit together the curvatures of section 4 or 6 and zone 7, zones 7 and 8, and zone 8 and section 9, respectively. First and second derivatives of a profile in the transition zones can be continuous, but the boundaries or transition zones 7a and 11 may also comprise a bend.

A transition zone 10 is present in a radial direction between section 4 and the additional posterior surface section comprising both zones 7 and 8 in the embodiment of figure 7. A profile for such a transition has been disclosed in WO 2010/095938, which is incorporated herein by reference. In an embodiment the transition zone (10) is shaped such as to refract light away from optical axis R1 when IOL 1 is illuminated via a collimated lens and placed in an ISO model eye having a 4 mm diameter aperture. In an embodiment transition zone 10 has a curvature resulting in a loss of light within a circle with a diameter of 4 mm around said optical axis of less than about 25%, the loss of light being defined as the fraction of the amount of in-focus light from IOL 1 compared to the amount of light from an identical IOL without the additional posterior surface section.

An opening angle α between both transition zones 10, which cross at about optical axis R1 or intersect with optical axis R1 can be between 120 and 240 degrees, in an embodiment between 170 and 190 degrees, and is an opening angle of the additional posterior surface section comprising the two section zones or section parts 7 and 8. Figure 7 further shows two markers 50 to determine the position of the additional posterior surface section.

Figure 8 shows another embodiment. Most parts are equal to the ones described for the embodiment shown in figures 4 to 7, including the circumferential shape of the additional posterior surface section that includes just one section zone 17 in the present embodiment. Both embodiments are therefore identical, except for the additional posterior surface section. In the embodiment shown in figure 8 the additional posterior surface section comprises two parts that are superimposed within one section zone 17.

Section zone 17 comprises a first part that is configured for either near or intermediate vision. Its curvature is also embedded or recessed within the curvature of substantially concave main posterior surface 4. In general, the curvature of the first part of zone 17 is more flat then the curvature of main posterior surface 4. Its maximum optical power can be between +1.5 and +6.0 dioptre, in an embodiment between +1.50 and +4.0 dioptre, and is obtained by refraction from a base surface curvature of the first part. The refractive optical power may vary according to a specific profile across section 17. It can vary in a radial direction, and can both increase and decrease in that direction. The variation of the optical power across section zone 17 can be between 0 and 100% of the maximum refractive optical power of the first section part. Again, the optical power of section zone 17 is relative to the base optical power.

Section zone 17 further comprises a second part consisting of a diffractive structure superimposed on the base surface curvature of the first part. The maximum diffractive optical power addition to the refractive optical power of the first part is between +1.00 and +4.0 dioptre and can vary according to a specific profile across section zone 17. It can both increase and decrease in a radial direction. The diffractive structure shown in figure 8 is very much exaggerated. In practice, the diffractive feature will generally be 0.5 to 2 micron in size. Such a diffractive element is also disclosed with reference to figures 12 to 16 in WO 2010/095938, which is incorporated herein by reference, in which the diffractive pattern is superimposed on a convex surface whereas it is superimposed on a concave recessed surface for the present invention. Such a diffractive pattern is further disclosed in EP 0 888 564 and EP 1 194 797, which are incorporated herein by reference.

The optical power of the section zone 17 is therefore an addition of the optical powers of the first and second parts within section zone 17, which first and second parts are superimposed within the section zone. Section zone 17 is bifocal and optic 3 as a whole, including the main lens, is trifocal. Boundaries and transition zones 6a, 10 and 11 can be configured as disclosed for the embodiments of figures 4 to 7.

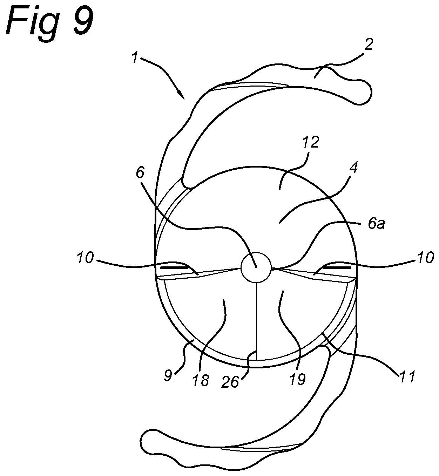

Figure 9 shows the back side of yet another embodiment of an IOL according to the invention. As for the previous embodiment, most parts are equal to the ones described for the embodiment shown in figures 4 to 7, including the circumferential shape of the additional posterior surface section. The additional posterior surface section includes two section zones or section parts 18 and 19 in the present embodiment. The embodiments of figures 4-7, of figure 8 and of figure 9 are therefore identical, except for the additional posterior surface section. In the embodiment shown in figure 9 the additional posterior surface section comprises two section zones 18 and 19 that are arranged angularly around optical axis R1, whereas the two section zones 8 and 9 of figure 7 are arranged concentric around optical axis R1.

The optical configuration of section zones can be as has been described for section zones 8 and 9, each section zone having, for instance, its own maximum optical power and a variation of the optical power across the section zone according to some specific profile. Again, the optical power may both increase and decrease in the radial direction. One of the section zones will generally be optimized for near vision, i.e. for reading purposes, and the other section zone for intermediate vision, while the main lens will generally be configured for distance vision. Section zone 18 and 19 is bifocal and optic 3 as a whole, including the main lens, is trifocal. The curvature of sector zones 18 and 19 will be more flat then a curvature of main posterior surface section 4.

Section zones 18 and 19 are physically divided by a boundary or transition zone 26, which can be configured as has been described with reference to boundaries or transition zones 6a, 7a and 11. Boundaries or transition zones 6a, 7a, 10, 11 and 26 are shown as lines in figures 7, 8 and 9, but need not be visible in an actual product. They are drawn as an indication only.

Each section zone 18 and 19 encloses an angle β and γ, respectively, around optical axis R1. Each angle β and γ will be between 90 and 150 degrees, in an embodiment between 100 and 130 degrees. The total enclosed angle of the posterior surface section, β+γ, will be between 120 and 240 degrees, in an embodiment between 170 and 190 degrees.

Figures 10a and 10b show a ray trace RT near the circumferential edge or rim 3 a of the IOL, where a steep transition is present from the recessed additional surface section to an (imaginary) surface of the main surface section. Figure 10a shows the additional surface section present on the posterior surface 12 of an IOL according to the invention. The additional surface section is recessed with respect to an imaginary extension 4a of the main posterior surface section. The ray trace RT of figure 10a is refracted such at the surfaces of the IOL that is directed away from the optical axis R1 of the IOL and therefore away from the optical axis of the eye when the IOL is positioned within the human eye.

Figure 10b shows an additional surface section present on an anterior surface 5 of a known IOL. The additional surface section is recessed with respect to an imaginary extension 4'a of a main anterior surface section The ray trace RT of figure 10b is internally reflected at the rim 3 a of the IOL, which results in the ray trace being directed towards the optical axis R1 of the IOL. The ray trace will therefore be directed towards the optical axis R100 of the human eye when the IOL is positioned in the human eye. This results in actual light passing through such transition between additional and main surface sections being directed towards the optical axis R100 of the human eye, which results in an undesirable halo observed.

Having the additional surface section on the posterior surface is therefore advantageous, since it does not present such a halo.

## Claims

1. IOL (intraocular lens) (1) for insertion into the human eye (100), said IOL comprising an optic (3), said optic comprising:
- a posterior surface (12) for facing towards the posterior chamber (107) of the human eye (100);
- an anterior surface (5) for facing away from the posterior chamber (107) of the human eye (100), said anterior surface being at least substantially convex;
- a main lens formed by corresponding surface sections of said anterior and posterior surfaces, said main lens having an optical axis (R1) and a base optical power; and
- an additional surface section at a distance from said optical axis, said additional surface section comprising at least one additional section part (7, 8, 18, 19), the or each additional section part providing for an optical power different from said base optical power,
**characterized in that** said posterior surface (12) is substantially flat or at least substantially concave, and said posterior surface (12) comprises:
- a substantially flat or at least substantially concave main posterior surface section (4), said main lens being formed by said anterior surface (5) and said main posterior surface section (4); and
- said additional surface section providing an additional posterior surface section at a distance from said optical axis, said additional posterior surface section comprising said at least one additional section part providing at least one refractive posterior section part (7, 8, 18, 19), the or each refractive posterior section part providing for said optical power different from said base optical power.

2. IOL according to claim 1, wherein said additional refractive posterior surface section is at least substantially recessed with respect to said main posterior surface (4).

3. IOL according to any one of the preceding claims, wherein the anterior surface (5) is at least substantially smooth.

4. IOL according to any one of the preceding claims, wherein said main lens has a base optical power configured for correcting a refractive error in the eye for distance vision, said base optical power being between -15 and +15 dioptre, in an embodiment between -8 and +8 dioptre.

5. IOL according to claim 4, wherein the or each refractive posterior section part has an additional optical power configured for correcting near or intermediate vision, said additional optical power of said posterior section part being between 0 and +6 dioptre, in an embodiment between 0 and +4.0 dioptre, in an embodiment between +1.5 and +4.0 dioptre, relative to said base optical power.

6. IOL according to claim 5, wherein said additional optical power of said refractive posterior section part (8, 9) varies between a minimum and a maximum value throughout said posterior section part.

7. IOL according to any one of the preceding claims, wherein said additional posterior surface section comprises physically separated posterior section zones (7, 8, 18, 19).

8. IOL according to any one of the preceding claims, wherein said optic (3) comprises a central section (6) within a circumscribing circle (6a) having a diameter between 0.1 and 2 mm, said optical axis (R1) of said main lens passing through said central part, said central part (6) having an optical power between -2.0 and +2.0 dioptre, in an embodiment between -1.0 and +1.0 dioptre, relative to said base optical power.

9. IOL according to claim 8, wherein said optical axis (R1) of said main lens passes through a centre of said circumscribing circle (6a) of said central section (6).

10. IOL according to any one of the preceding claims, wherein a transition zone (10) between said main posterior surface (4) and said additional posterior surface section is shaped such as to refract light away from said optical axis (R1) when said IOL (1) is illuminated via a collimated lens and placed in an ISO model eye having a 4 mm diameter aperture, in an embodiment said transition zone (10) having a curvature resulting in a loss of light within a circle with a diameter of 4 mm around said optical axis of less than about 25%, said loss of light being defined as the fraction of the amount of in-focus light from said IOL (1) compared to the amount of light from an identical IOL without said additional posterior surface section.

11. IOL according to any one of the preceding claims, wherein said optical axis (R1) is directed such that that said optical axis (R1) is substantially aligned with an optical axis (R100) of the human eye (100) when said IOL is provided in the human eye.

12. Add-on IOL for insertion into the human eye in addition to the natural eye lens (106) or in addition to a replacement IOL (200) replacing the natural eye lens, said add-on IOL being an IOL as described in any one of the preceding claims.

13. Add-on IOL according to claim 12 for insertion in the sulcus (110) between the iris (104) and the capsular bag (109); or in the anterior chamber (103).

14. Add-on IOL according to claim 13 for insertion in the sulcus (110), said IOL comprising haptics (2) for holding said IOL within the human eye, said haptics enclosing an angle with a plane through a rim (3a) of said optic (3) such that said haptics are at a side of said plane corresponding to said anterior surface (5) of said IOL, in an embodiment an absolute value of said angle being between 5 and 15 degrees, in an embodiment between 8 and 12 degrees.

15. Add-on IOL according to claim 13 for insertion in the anterior chamber (103), said IOL comprising haptics (2) for holding said IOL within the human eye, said haptics enclosing an angle with a plane through a rim (3a) of said optic (3) such that said haptics are at a side of said plane corresponding to said posterior surface (12) of said IOL, in an embodiment an absolute value of said angle being between 5 and 15 degrees, in an embodiment between 8 and 12 degrees.

## Patentansprüche

1. IOL (Intraokularlinse) (1) zum Einführen in das menschliche Auge (100), wobei die IOL eine Optik (3) aufweist, wobei die Optik Folgendes aufweist:
- eine hintere Oberfläche (12), die zur hinteren Kammer (107) des menschlichen Auges (100) hinweist;
- eine vordere Oberfläche (5), die von der hinteren Kammer (107) des menschlichen Auges (100) weg weist, wobei die vordere Oberfläche zumindest im Wesentlichen konvex ist;
- eine Hauptlinse, die durch entsprechende Oberflächenabschnitte der vorderen und hinteren Oberflächen gebildet wird, wobei die Hauptlinse eine optische Achse (R1) und eine optische Grundleistung aufweist; und
- einen zusätzlichen Oberflächenabschnitt in einem Abstand von der optischen Achse, wobei der zusätzliche Oberflächenabschnitt mindestens einen zusätzlichen Abschnittsteil (7, 8, 18, 19) aufweist, wobei der oder jeder zusätzliche Abschnittsteil eine von der optischen Grundleistung unterschiedliche optische Leistung liefert,
**dadurch gekennzeichnet, dass** die hintere Oberfläche (12) im Wesentlichen flach oder zumindest im Wesentlichen konkav ist und die hintere Fläche (12) Folgendes aufweist:
- einen im Wesentlichen flachen oder zumindest im Wesentlichen konkaven hinteren Hauptoberflächenabschnitt (4), wobei die Hauptlinse durch die vordere Oberfläche (5) und den hinteren Hauptoberflächenabschnitt (4) ausgebildet ist; und
- wobei der zusätzliche Oberflächenabschnitt einen zusätzlichen hinteren Oberflächenabschnitt in einem Abstand von der optischen Achse vorsieht, wobei der zusätzliche hintere Oberflächenabschnitt den mindestens einen zusätzlichen Abschnittsteil aufweist, der mindestens einen lichtbrechenden bzw. refraktären hinteren Abschnittsteil (7, 8, 18, 19) vorsieht, wobei der oder jeder refraktäre hintere Abschnittsteil die optische Leistung, die sich von der optischen Basisleistung unterscheidet, vorsieht.

2. IOL nach Anspruch 1, wobei der zusätzliche refraktäre hintere Oberflächenabschnitt zumindest im Wesentlichen in Bezug auf die hintere Hauptoberfläche (4) ausgenommen bzw. vertieft ist.

3. IOL nach einem der vorhergehenden Ansprüche, wobei die vordere Oberfläche (5) zumindest im Wesentlichen glatt ist.

4. IOL nach einem der vorhergehenden Ansprüche, wobei die Hauptlinse eine optische Grundleistung aufweist, die konfiguriert ist zum Korrigieren eines Brechungsfehlers im Auge für die Fernsicht, wobei die optische Grundleistung zwischen -15 und +15 Dioptrien liegt, in einer Ausführungsform zwischen -8 und +8 Dioptrien.

5. IOL nach Anspruch 4, wobei der oder jeder refraktäre hintere Abschnittsteil eine zusätzliche optische Leistung aufweist, die konfiguriert ist zur Korrektur von Nahsicht oder Intermediärsicht, wobei die zusätzliche optische Leistung des hinteren Abschnittsteils zwischen 0 und +6 Dioptrien liegt, in einer Ausführungsform zwischen 0 und +4,0 Dioptrien, in einer Ausführungsform zwischen +1,5 und +4,0 Dioptrien, und zwar bezogen auf die optische Grundleistung.

6. IOL nach Anspruch 5, wobei die zusätzliche optische Leistung des refraktären hinteren Teilabschnitts (8, 9) zwischen einem minimalen und einem maximalen Wert über dem gesamten hinteren Abschnittsteil variiert.

7. IOL nach einem der vorhergehenden Ansprüche, wobei der zusätzliche hintere Oberflächenabschnitt physisch getrennte hintere Abschnittszonen (7, 8, 17, 18, 19) aufweist.

8. IOL nach einem der vorhergehenden Ansprüche, wobei die Optik (3) einen zentralen Abschnitt (6) innerhalb eines umschreibenden Kreises (6a) mit einem Durchmesser zwischen 0,1 und 2 mm aufweist, wobei die optische Achse (R1) der Hauptlinse durch den zentralen Teil verläuft, wobei der zentrale Teil (6) eine optische Leistung zwischen -2,0 und +2,0 Dioptrien hat, in einer Ausführungsform zwischen -1,0 und +1,0 Dioptrien, und zwar bezogen auf die optische Grundleistung.

9. IOL nach Anspruch 8, wobei die optische Achse (R1) der Hauptlinse durch eine Mitte des umschreibenden Kreises (6a) des zentralen Abschnitts (6) verläuft.

10. IOL nach einem der vorhergehenden Ansprüche, wobei eine Übergangszone (10) zwischen der hinteren Hauptoberfläche (4) und dem zusätzlichen hinteren Oberflächenabschnitt so geformt ist, dass sie Licht von der optischen Achse (R1) weg bricht, wenn die IOL (1) über eine kollimierte Linse beleuchtet und in ein ISO-Modellauge mit einer Öffnung von 4 mm Durchmesser angeordnet wird, wobei in einer Ausführungsform die Übergangszone 10 eine Krümmung hat, was in einem Lichtverlust innerhalb eines Kreises mit einem Durchmesser von 4 mm um die optische Achse herum von weniger als etwa 25% resultiert, wobei der Lichtverlust als der Bruchteil der Menge an In-Fokus-Licht von der IOL (1) im Vergleich zu der Lichtmenge von einer identischen IOL ohne den zusätzlichen hinteren Oberflächenabschnitt definiert ist.

11. IOL nach einem der vorhergehenden Ansprüche, wobei die optische Achse (R1) so gerichtet ist, dass die optische Achse (R1) im Wesentlichen mit einer optischen Achse (R100) des menschlichen Auges (100) ausgerichtet ist, wenn die IOL in dem menschlichen Auge vorgesehen ist.

12. Add-on-IOL zum Einführen in das menschliche Auge zusätzlich zu der natürlichen Augenlinse (106) oder zusätzlich zu einer Ersatz-IOL (200), die die natürliche Augenlinse ersetzt, wobei die Add-on-IOL eine IOL ist, wie sie in einem der vorangegangenen Ansprüche beschrieben ist.

13. Add-on IOL nach Anspruch 12 zum Einführen in den Sulcus (110) zwischen der Iris (104) und dem Kapselsack (109); oder in die vordere Kammer (103).

14. Add-on IOL nach Anspruch 13 zum Einführen in den Sulcus (110), wobei die IOL Haptikelemente (2) aufweist zum Halten der IOL innerhalb des menschlichen Auges, wobei die Haptikelemente einen Winkel mit einer Ebene durch einen Rand (3a) der Optik (3) einschließen, und zwar derart, dass die Haptikelemente an einer der vorderen Oberfläche (5) der IOL entsprechenden Seite der Ebene liegen, wobei in einer Ausführungsform ein Absolutwert des Winkels zwischen 5 und 15 Grad liegt, in einer Ausführungsform zwischen 8 und 12 Grad.

15. Add-on-IOL nach Anspruch 13 zum Einführen in die vordere Kammer (103), wobei die IOL Haptikelemente (2) zum Halten der IOL innerhalb des menschlichen Auges aufweist, wobei die Haptikelemente einen Winkel mit einer Ebene durch einen Rand (3a) der Optik (3) einschließen, und zwar derart, dass die Haptikelemente an einer der hinteren Oberfläche (12) der IOL entsprechenden Seite der Ebene liegen, wobei in einer Ausführungsform ein Absolutwert des Winkels zwischen 5 und 15 Grad liegt, in einer Ausführungsform zwischen 8 und 12 Grad.

## Revendications

1. LIO (lentille intraoculaire) (1) destinée à être insérée dans un oeil humain (100), ladite LIO comprenant une optique (3), ladite optique comprenant :
- une surface postérieure (12) destinée à être orientée face à la chambre postérieure (107) d'un oeil humain (100) ;
- une surface antérieure (5) destinée à être orientée à l'opposé de la chambre postérieure (107) de l'oeil humain (100), ladite surface antérieure étant au moins sensiblement convexe ;
- une lentille principale formée par des sections de surface correspondantes desdites surfaces antérieures et postérieures, ladite lentille principale présentant un axe optique (R1) et une puissance optique de base ; et
- une section de surface supplémentaire à distance dudit axe optique, ladite section de surface supplémentaire comprenant au moins une partie de section supplémentaire (7, 8, 18, 19), la ou chaque partie de section supplémentaire apportant une puissance optique différente de ladite puissance optique de base,
**caractérisée en ce que** ladite surface postérieure (12) est sensiblement plane ou au moins sensiblement concave, et ladite surface postérieure (12) comprend :
- une section de surface postérieure principale sensiblement plane ou au moins sensiblement concave (4), ladite lentille principale étant formée par ladite surface antérieure (5) et ladite section de surface postérieure principale (4) ; et
- ladite section de surface supplémentaire offrant une section de surface postérieure supplémentaire à distance dudit axe optique, ladite section de surface postérieure supplémentaire comprenant ladite au moins une partie de section supplémentaire offrant au moins une partie de section postérieure réfractive (7, 8, 18, 19), la ou chaque partie de section postérieure réfractive apportant ladite puissance optique différente de ladite puissance optique de base.

2. LIO selon la revendication 1, dans laquelle ladite section de surface postérieure réfractive supplémentaire est au moins sensiblement en retrait par rapport à ladite surface postérieure principale (4).

3. LIO selon l'une quelconque des revendications précédentes, dans laquelle la surface antérieure (5) est au moins sensiblement lisse.

4. LIO l'une quelconque des revendications précédentes, dans laquelle ladite lentille principale présente une puissance optique de base configurée pour corriger une erreur de réfraction dans l'oeil pour la vision à distance, ladite puissance optique de base étant comprise entre -15 et +15 dioptries, dans un mode de réalisation entre -8 et +8 dioptries.

5. LIO selon la revendication 4, dans laquelle la ou chaque partie de section postérieure réfractive présente une puissance optique supplémentaire configurée pour corriger la vision proche ou intermédiaire, ladite puissance optique supplémentaire de ladite partie de section postérieure étant comprise entre 0 et +6 dioptries, dans un mode de réalisation entre 0 et +4,0 dioptries, dans un mode de réalisation entre +1,5 et +4,0 dioptries, par rapport à ladite puissance optique de base.

6. LIO selon la revendication 5, dans laquelle ladite puissance optique supplémentaire de ladite partie de section postérieure réfractive (8, 9) varie entre une valeur minimale et une valeur maximale à travers ladite partie de section postérieure.

7. LIO selon l'une quelconque des revendications précédentes, dans laquelle ladite section de surface postérieure supplémentaire comprend des zones de section postérieure physiquement séparées (7, 8, 18, 19).

8. LIO selon l'une quelconque des revendications précédentes, dans laquelle ladite optique (3) comprend une section centrale (6) inscrite dans un cercle (6a) présentant un diamètre compris entre 0.1 et 2 mm, ledit axe optique (R1) de ladite lentille principale passant à travers ladite partie centrale, ladite partie central (6) présentant une puissance optique comprise entre -2,0 et +2,0 dioptries, dans un mode de réalisation entre -1,0 et +1,0 dioptries, par rapport à ladite puissance optique de base.

9. LIO selon la revendication 8, dans laquelle l'axe optique (R1) de ladite lentille principale passe par le centre dudit cercle (6a) dans lequel s'inscrit ladite section centrale (6).

10. LIO selon l'une quelconque des revendications précédentes, dans laquelle une zone de transition (10) entre ladite surface postérieure principale (4) et ladite section de surface postérieure supplémentaire est conformée pour réfracter la lumière à l'opposé dudit axe optique (RI) quand ladite LIO (1) est éclairée par l'intermédiaire d'une lentille collimatrice et placée dans un modèle ISO d'oeil présentant une ouverture de 4 mm de diamètre, dans un mode de réalisation ladite zone de transition (10) présentant une courbure entraînant une perte de lumière à l'intérieur d'un cercle de 4 mm de diamètre autour dudit axe optique de moins de 25 % environ, ladite perte de lumière étant définie comme étant la fraction de la quantité de lumière focalisée à partie de ladite LIO (1) par rapport à la quantité de lumière en provenance d'une LIO identique sans ladite section de surface postérieure supplémentaire.

11. LIO selon l'une quelconque des revendications précédentes, dans laquelle ledit axe optique (R1) est orienté de manière à ce que ledit axe optique (R1) soit sensiblement aligné avec un axe optique (R100) de l'oeil humain (100) quand ladite LIO est prévue dans un oeil humain.

12. LIO complémentaire destinée à être insérée dans un oeil humain en plus de la lentille naturelle de l'oeil (106) ou en plus d'une LIO de remplacement (200) remplaçant la lentille naturelle de l'oeil, ladite LIO complémentaire étant une LIO telle que décrite dans l'une quelconque des revendications précédentes.

13. LIO complémentaire selon la revendication 12 destinée à être insérée dans le sillon (110) entre l'iris (104) et le sac capsulaire (109) ; ou dans la chambre antérieure (103).

14. LIO complémentaire selon la revendication 13 destinée à être insérée dans le sillon (110), ladite LIO comprenant des haptiques (2) pour maintenir ladite LIO dans l'oeil humain, lesdites haptiques formant un angle par rapport à un plan traversant un rebord (3a) de ladite optique (3) de sorte que lesdites haptiques se trouvent d'un côté dudit plan correspondant à ladite surface antérieure (5) de ladite LIO, dans un mode de réalisation une valeur absolue dudit angle étant comprise entre 5 et 15 degrés, et dans un mode de réalisation entre 8 et 12 degrés.

15. LIO complémentaire selon la revendication 13 destinée à être insérée dans la chambre antérieure (103), ladite LIO comprenant des haptiques (2) pour maintenir ladite LIO dans l'oeil humain, lesdites haptiques formant un angle par rapport à un plan traversant un rebord (3a) de ladite optique (3) de sorte que lesdites haptiques se trouvent d'un côté dudit plan correspondant à ladite surface postérieure (12) de ladite LIO, dans un mode de réalisation une valeur absolue dudit angle étant comprise entre 5 et 15 degrés, et dans un mode de réalisation entre 8 et 12 degrés.
